# EUROPEAN PATENT SPECIFICATION

(11) **EP 4 323 055 B1**
(45) Date of publication and mention of the grant of the patent: **12.08.2026**
(21) Application number: 21721410.5
(22) Date of filing: 16.04.2021
(51) Int. Cl.: A61N 1/36, H04R 25/00

(54) **HEARING PROSTHESIS SYSTEM**
HÖRPROTHESENSYSTEM
SYSTÈME DE PROTHÈSE AUDITIVE

(43) Date of publication of application: 21.02.2024
(73) Proprietor: Advanced Bionics AG, 8712 Staefa (CH)
(72) Inventor: FREDELAKE, Stefan, 30625 Hannover (DE); KOHL, Manuel Christoph, 30655 Hannover (DE); CHALUPPER, Josef, 85307 Paunzhausen (DE)
(74) Representative: Schwan Schorer & Partner mbB
(86) International application number: PCT/EP2021/059871
(87) International publication number: WO 2022/218538

(56) References cited:
- US-A1- 2013 006 044
- US-A1- 2015 377 832
- US-A1- 2018 296 845
- US-A1- 2018 352 349

## Description

The disclosure relates to an implantable hearing prosthesis system, such as a cochlear implant system, middle-ear implant system, bone conduction implant system, or auditory brain stem implant system.

Such implantable hearing prosthesis systems comprise an implantable part which is typically anchored inside the temporal bone of the patient and a head- or body-worn external electronic device which communicates with the implantable part via a transcutaneous short range radio frequency (RF) connection which is stablished by transcutaneous inductive coupling between an implant transceiver coil in the implantable part and an external transceiver coil in the external electronic device. To facilitate such transcutaneous connection, a separate headpiece with an integrated attachment magnet system may be provided which mechanically aligns itself to an attachment magnet system provided in the implantable part when the headpiece is placed in the vicinity of the implantable part.

Due to individual variations in anatomy, in particular with regard to the thickness of the skin flap between the hearing implant and the external headpiece, the magnetic retention force exhibited by the same magnet system may vary substantially across recipients and time. To address such individual variations, it is established practice by manufacturers of such systems to provide a graduated set of magnets with increasing strength insertable into the external magnet system for the implanting clinic to choose therefrom. Selection of a magnet with an appropriate strength is of great importance, since overly strong retention forces resulting from a too strong magnet may give rise to potentially severe compression-induced damage, such as inflammation, to the skin flap, whereas a too low retention force resulting from a too weak magnet may result in the headpiece losing its attachment to the implant magnet system and/or may result in the headpiece coming off the head without such action being intended by the patient. In the current practice, the person selecting the external magnet typically relies on subjective feedback from the patient like "it feels too loose", "it feels too firm" or "this is a comfortable / uncomfortable fit".

Moreover, the required optimum magnet strength may vary over time. For example, shortly after surgery, the skin flap might be swollen, resulting in an increased transcutaneous distance which requires a stronger magnet. Over time, the skin thickness might decrease due to the pressure exerted by the magnets, subsequently requiring a weaker magnet to be selected. Growing hair as well as varying haircuts may also result in different magnet strength requirements.

US 2018/0352349 A1 relates to a cochlear implant system, wherein the headpiece comprises a sensor for measuring the magnetic field strength of the implant magnet for estimating the thickness of the skin flap from the measured field strength and for automatically determining the required magnet strength. The headpiece may include a magnet that can be programmed or adjusted to the required strength.

US 2018/0296845 A1 relates to a cochlear implant system, wherein the external part comprises a measurement unit connected to the external transceiver coil for measuring an electrical parameter like the RF signal amplitude. The measured values of the parameter are stored so as to detect a change in the electrical parameter, upon which the external part may be put into a low power mode.

WO 2004/021876 A1 relates to a cochlear implant system, wherein the strength of the magnetic field in the vicinity of the transceiver coil of the external part is measured so as to estimate the distance between the external transceiver coil and the implant transceiver coil. The measurement results may be used for estimating a skin-flap thickness.

WO 2019/046133 A1 and WO 2019/045681 A1 relate to a cochlear implant system, wherein the external headpiece generates an RSSI signal based on a signal strength of the detected wireless back telemetry signal received from the implant. The RSSI signal is used to present an indication of the a degree of alignment of the headpiece with the implant to assist a user in aligning the headpiece with the implant.

US 7,450,994 B1 relates to a cochlear implant system, wherein thickness of a skin flap is estimated by measuring an amount of electrical energy stored in the implant while varying a stimulation load signal and/or a power level applied to the implant, and wherein the collected measurement data is compared with predetermined calibration data obtained prior to implantation by using flap simulators.

US 2015/377832 A1 relates to a diagnostic device for selecting the proper external magnet of a cochlear implant system based on measurements of the implant magnet system, wherein the diagnostic device is placed on the patient's skin substantially over the implant with the implant magnet after implantation. The diagnostic device comprises a primary sensor for measuring a characteristic of the magnetic field of the implanted magnet, such as a strength of the magnetic field or a polarity of the magnetic field. The diagnostic device further comprises navigation sensors which measure a strength of the magnetic field and provide for a navigation signal including information indicative of the measured strength; one of the navigation sensors may be a sensor configured to measure a distance of the diagnostic device and/or the primary sensor to the patient's skin.

It is an objective of the disclosure to overcome the drawbacks of the prior art and to provide for an implantable hearing prosthesis system which allows to improve magnetic attachment of the external part.

According to the invention, the objective is achieved by a system as defined in claim 1.

In some embodiments the disclosure suggests to measure coil coupling strength values indicative of the received coil telemetry coupling strength when the external transceiver coil and the implant transceiver coil are inductively coupled and to subsequently analyze the measured values. Thereby an estimation of a present transcutaneous distance and/or an estimation of a present transcutaneous magnetic coupling strength between the implant attachment magnet and the external attachment magnet and/or a target value of a desired transcutaneous magnetic coupling strength can be obtained in a relatively simple and reliable manner.

In particular, since the transceiver circuitry used for operating the external transceiver coil typically anyway is configured to measure a received coil telemetry coupling strength, no hardware modification of the external part is required for implementing the proposed analyzing results.

In some implementations, the analyzing unit may be implemented in a device which is separate from the external component and which is configured to be communicatively coupled to the external part, such as a fitting device of the audiologist or a mobile phone or tablet computer of the patient.

Some embodiments of the disclosure are defined in the dependent claims.

Hereinafter, examples of the disclosure will be illustrated by reference to the attached drawings, wherein:
Fig. 1 is a schematic representation of an example of a hearing prosthesis system according to the disclosure;
Fig. 2 is a perspective illustration of an example of a cochlear implant system according to the disclosure;
Fig. 3 is a perspective illustration of an example of the external components of the system of Fig. 2;
Fig. 4 is a perspective illustration of an example of the implantable components of the system of Fig. 2;
Fig. 5Ashows an example of coil coupling strength values measured by a hearing prosthesis system during a plurality of headpiece attachment actions; and Fig. 5B illustrates an average of the measurements of Fig. 5A including error bars, wherein magnet retention is satisfactory;
Figs. 6A and 6B are illustrations like Figs. 5A and 5B, respectively, wherein magnet retention is too weak;
Figs. 7A and 7B are illustrations like Figs. 5A and 5B, respectively, wherein magnet retention is too strong; and
Fig. 8A shows an example of a relationship of the distance between an implant coil and an external coil of a hearing prosthesis system and measured MTEL-RSSI values, and Fig. 8B shows an average / median of the values of Fig. 8A.

The drawings have not necessarily been drawn to scale. Similarly, some components and/or operations may be separated into different blocks or combined into a single block for the purposes of discussion of some of the embodiments of the disclosure. Moreover, while the disclosure is amenable to various modifications and alternative forms, specific embodiments have been shown by way of example in the drawings and are described in detail below. The intention, however, is not to limit the invention to the particular embodiments described. On the contrary, the invention is intended to cover all modifications, and alternatives falling within the scope of the invention as defined by the appended claims.

As used hereinafter, an "attachment magnet device" is any magnetic arrangement that is configured to facilitate attachment of an external component at the body, in particular at the head, of the recipient via transcutaneous magnetic coupling. For example, an "attachment magnet device" may be formed by a single piece of a permanent magnetic material, or it may include a plurality of magnetically interacting pieces of a permanent magnetic material. The magnet piece(s) may be fixed within a housing, or may be movable, in particular, rotatable, within the housing.

As used hereinafter, an "external component" of a hearing prosthesis system may be any external unit which includes an attachment magnet device for attachment at the body, in particular at the head, of the recipient via transcutaneous magnetic coupling to an implant attachment magnet device and which includes a transceiver coil for inductive coupling with an implant transceiver coil. In some implementations, the external component may be a headpiece connected to a sound processor, and in other implementations it may headpiece which includes sound processor functionality.

As used hereinafter, an "average" is the result of any type of averaging procedure, such as any kind of mean (e.g., arithmetic mean, geometric mean, harmonic mean, interquartile mean, weighted mean), mid-range, median, etc.

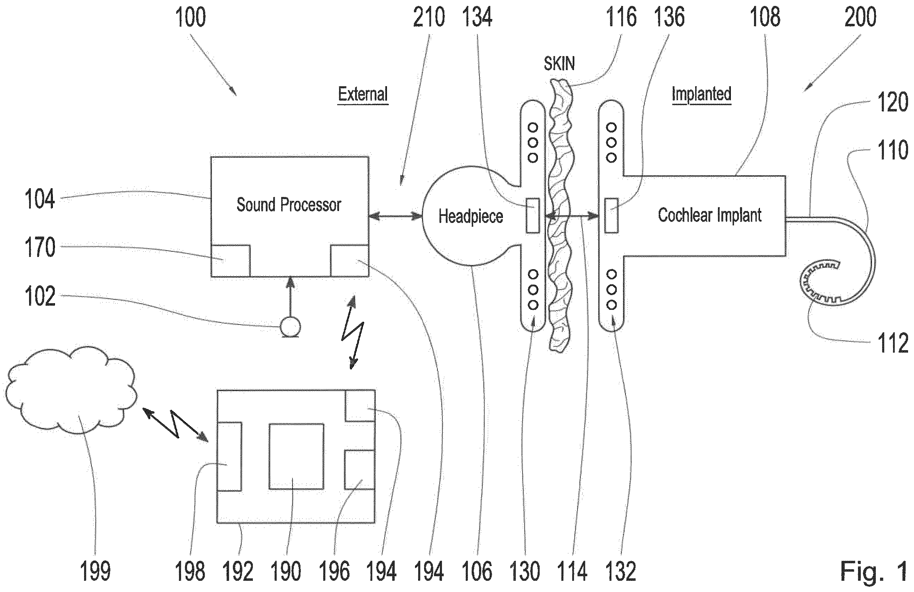

FIG. 1 illustrates an implantable hearing prosthesis system, which in the illustrated example is implemented as a cochlear implant system 100. While the specific examples shown hereinafter relate to cochlear implant systems, the present disclosure also is applicable in other types of hearing prosthesis systems, such as middle-ear implant systems or auditory brain stem implant systems. The present disclosure is generally applicable to implantable systems which comprise attachment magnet devices and inductively coupled transceiver coils.

As shown, cochlear implant system 100 may include a microphone 102, a sound processor 104, a headpiece 106 having a coil disposed therein, a cochlear implant 108, and an electrode lead 110.

Electrode lead 110 includes an array of stimulating electrodes 112 disposed on a distal portion of electrode lead 110 and that are configured to be located within and to stimulate the cochlea after the distal portion of electrode lead 110 is inserted into the cochlea. As shown, electrode lead 110 may be pre-curved so as to properly fit within the spiral shape of the cochlea. Additional or alternative components may be included within cochlear implant system 100 as may serve a particular implementation.

As shown, cochlear implant system 100 may include various components configured to be located external to a patient including, but not limited to, microphone 102, sound processor 104, and headpiece 106. Cochlear implant system 100 may further include various components configured to be implanted within the patient including, but not limited to, cochlear implant 108 and electrode lead 110.

Microphone 102 may be configured to detect audio signals presented to the user. Microphone 102 may be implemented in any suitable manner. For example, microphone 102 may include a microphone that is configured to be placed within the concha of the ear near the entrance to the ear canal, such as a T-MIC^{™} microphone from Advanced Bionics. Such a microphone may be held within the concha of the ear near the entrance of the ear canal by a boom or stalk that is attached to an ear hook configured to be selectively attached to sound processor 104. Additionally or alternatively, microphone 102 may be implemented by one or more microphones disposed within headpiece 106, one or more microphones disposed within sound processor 104, one or more beam-forming microphones, and/or any other suitable microphone as may serve a particular implementation.

Sound processor 104 (i.e., one or more components included within sound processor 104) may be configured to direct cochlear implant 108 to generate and apply electrical stimulation (also referred to herein as "stimulation current") representative of one or more audio signals (e.g., one or more audio signals detected by microphone 102, input by way of an auxiliary audio input port, input by way of a clinician's programming interface ("CPI") device, etc.) to one or more stimulation sites associated with an auditory pathway (e.g., the auditory nerve) of the patient. Exemplary stimulation sites include, but are not limited to, one or more locations within the cochlea, the cochlear nucleus, the inferior colliculus, and/or any other nuclei in the auditory pathway. To this end, sound processor 104 may process the one or more audio signals in accordance with a selected sound processing strategy or program to generate appropriate stimulation parameters for controlling cochlear implant 108. Sound processor 104 may be housed within any suitable housing (e.g., a behind-the-ear ("BTE") unit, a body worn device, headpiece 106, and/or any other sound processing unit as may serve a particular implementation).

In some examples, sound processor 104 may wirelessly transmit stimulation parameters (e.g., in the form of data words included in a forward telemetry sequence) and/or power signals to cochlear implant 108 by way of a wireless communication link 114 between headpiece 106 and cochlear implant 108. It will be understood that communication link 114 may include a bi-directional communication link and/or one or more dedicated uni-directional communication links.

Headpiece 106 may be communicatively coupled to sound processor 104 and includes an external transceiver coil 130 configured to facilitate selective wireless coupling of sound processor 104 to cochlear implant 108. Headpiece 106 may additionally or alternatively be used to selectively and wirelessly couple any other external device to cochlear implant 108. To this end, headpiece 106 may be configured to be affixed to the patient's head and positioned such that the external antenna housed within headpiece 106 is communicatively coupled to a corresponding implantable transceiver coil 132 included within or otherwise associated with cochlear implant 108. In this manner, stimulation parameters and/or power signals may be wirelessly transmitted between sound processor 104 and cochlear implant 108 via a communication link 114.

To facilitate retention of the headpiece 106 at the head and to align the transceiver coils 130, 132 relative to the each other for allowing optimal inductive coupling, the headpiece 106 comprises an external attachment magnet device 134 and the cochlear implant 108 comprises an implant attachment magnet device 136. The attachment devices may be located in the central of the respective coil 130, 132, so as to optimize alignment of the coils 130, 132. The magnetic coupling between the external attachment magnet device 134 and the implant attachment magnet device 136 results in magnetic attraction / coupling forces which press the headpiece 106 against the skin 116 above the cochlear implant 108. Each of the magnet devices 134, 136 may consist of a single magnet of may comprise a plurality of interacting magnets. The magnets may be fixed within the headpiece 106 and the cochlear implant, or they may be moveable, in particular rotatable around a single axis or a plurality of axes, so as to enhance MRI compatibility. In other implementations, the magnet(s) may be removable for replacement and/or for enhancing MRI compatibility. Examples of magnet attachment devices which may be used with the present disclosure are described, e.g., in US 9,919,154 B2.

Cochlear implant 108 may include any type of implantable stimulator that may be used in association with the systems and methods described herein. For example, cochlear implant 108 may be implemented by an implantable cochlear stimulator. In some alternative implementations, cochlear implant 108 may include a brainstem implant and/or any other type of cochlear implant that may be implanted within a patient and configured to apply stimulation to one or more stimulation sites located along an auditory pathway of a patient.

In some examples, cochlear implant 108 may be configured to generate electrical stimulation representative of an audio signal processed by sound processor 104 (e.g., an audio signal detected by microphone 102) in accordance with one or more stimulation parameters transmitted thereto by sound processor 104. Cochlear implant 108 may be further configured to apply the electrical stimulation to one or more stimulation sites (e.g., one or more intracochlear regions) within the patient via electrodes 112 disposed along electrode lead 110 (e.g., applying current by way of stimulating electrodes 112 that returns by way of a ground electrode 120). In some examples, cochlear implant 108 may include a plurality of independent current sources each associated with a channel defined by one or more of electrodes 112. In this manner, different stimulation current levels may be applied to multiple stimulation sites simultaneously by way of multiple electrodes 112.

The human cochlea is in the shape of a spiral beginning at a base and ending at an apex, and auditory nerve tissue resides within the cochlea resides. The auditory nerve tissue is organized within the cochlea in a tonotopic manner. Relatively low frequencies are encoded at or near the apex of the cochlea (referred to as an "apical region") while relatively high frequencies are encoded at or near the base (referred to as a "basal region"). Hence, electrical stimulation applied by way of electrodes disposed within the apical region (i.e., "apical electrodes") may result in the patient perceiving relatively low frequencies and electrical stimulation applied by way of electrodes disposed within the basal region (i.e., "basal electrodes") may result in the patient perceiving relatively high frequencies. The delineation between the apical and basal electrodes on a particular electrode lead may vary depending on the insertion depth of the electrode lead, the anatomy of the patient's cochlea, and/or any other factor as may serve a particular implementation.

The headpiece 106 with the external transceiver coil 130 and the external attachment magnet device 134 is an example of an "external component", and the cochlear implant 108 with the implant transceiver coil 132 and the implant attachment magnet device 136 is an example of a "hearing implant", as used hereinafter.

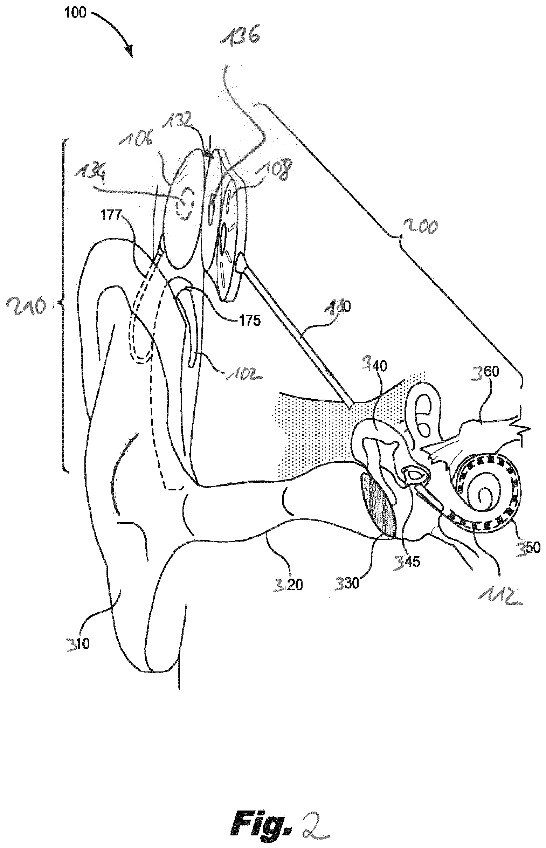

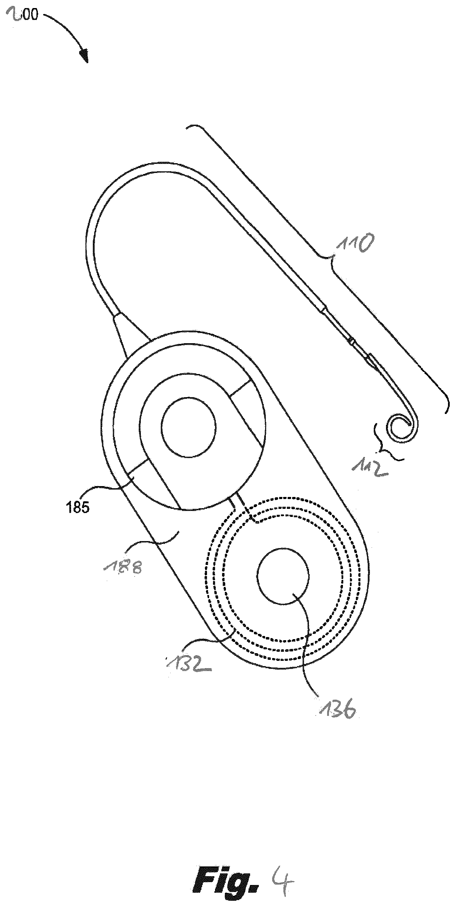

An example of an implementation of the cochlear implant system 100 of Fig. 1 is illustrated in Figs. 2 to 4, wherein a cochlear implant system 100 is shown which comprises an implantable portion 200, including the cochlea implant 108 and the electrode array 110, and an external portion 210, including the microphone 102, the sound processor 104 and the headpiece 106, wherein the implantable portion 200 has been implanted within a patient's head. As a result, the electrode array 112 has been surgically placed within the patient's cochlea 350.

In a properly functioning human ear, sound enters the pinna 310 and is directed into the ear canal 320, where the sound wave vibrates the tympanic membrane 330. The motion of the tympanic membrane 330 is amplified and transmitted through the ossicular chain 340 which includes three bones in the middle ear. The third bone of the ossicular chain 340, the stapes 345, contacts the outer surface of the cochlea 350 and causes movement of the fluid within the cochlea 350. Cochlear hair cells respond to the fluid-borne vibration and trigger neural electrical signals that are conducted from the cochlea 350 to the auditory cortex via the auditory nerve 360.

The cochlear implant system 100 operates by direct electrical stimulation of the auditory nerve cells, bypassing the defective cochlear hair cells that normally transduce acoustic energy into electrical energy.

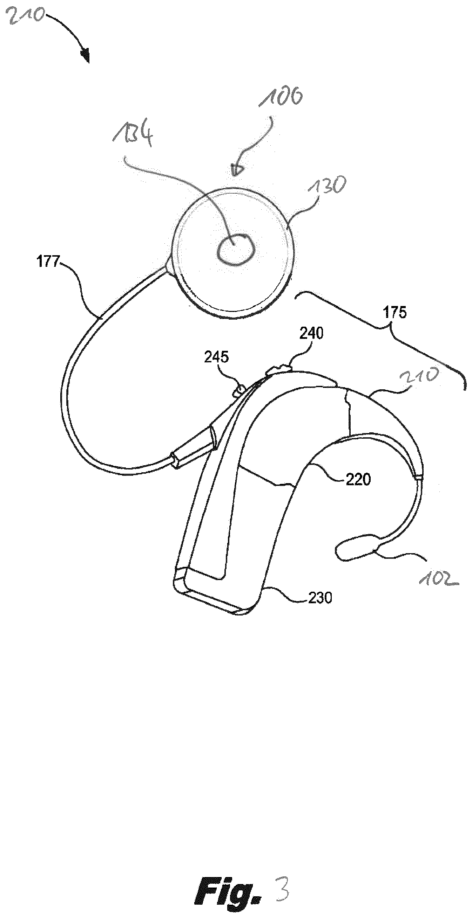

The external portion 210 of the cochlear implant system 100 is shown in Fig. 3, wherein the sound processor 104 is implemented as a behind-the-ear (BTE) unit 175 which comprises the microphone 102, an ear hook 215, sound processing circuitry 220 and a battery 230, which may be rechargeable. The audio signals picked up by the microphone 102 are processed by the sound processing circuitry 220, and the processed electrical signals are sent through a cable 177 to the headpiece 106 with the transceiver coil 130. A number of controls 240, 245 may adjust the operation of the sound processing circuitry 320 and may include a volume switch 340 and a program selection switch 245. The processed electrical signals from the sound processing circuitry 320 and power from the battery 230 is inductively transmitted to the implant transceiver coil 132 of the cochlea implant 108. The electrical signals and power received by the implant coil 133 are supplied to stimulation signal processing circuitry 185 of the cochlea implant 108, from where process stimulation signals are supplied through the electrode lead 110 to the stimulation electrode array 112 for electrical stimulation of the cochlea. The implant coil 136 and the stimulation signal processing circuitry 185 may be secured within an encapsulant 188 which may be made of medical grade silicone.

The external portion 210 of the cochlear implant system 100 comprises a measurement unit 170 for measuring coil coupling strength values indicative of the received coil telemetry coupling strength when the external transceiver coil 130 and the implant transceiver coil 132 are inductively coupled. Such measurement unit may be implemented, for example, in the headpiece 106 or in the sound processor 104. The received coil telemetry coupling strength (which also will be labeled "MTEL-RSSI" hereinafter) is a measure of the strength of the inductive coupling between the two coils 130, 132. Since the strength of the inductive coupling between the coils 130, 132 depends on the distance between the coils, the received coil telemetry coupling strength is also a measure of the transcutaneous distance between the implant coil 132 and the external coil 130.

The received coil telemetry coupling strength may be provided by the transceiver chip used in the sound processor 104 as a variable which can be easily read out from the sound processor 104. For example, the variable may be an array of the size [10x1]. As mentioned above, such variable correlates with the distance between the external coil 130 and the implant coil 132 and, since the coils 130, 132 are fixed within the headpiece 106 and the cochlea implant 108, respectively, such variable also correlates with the distance between the headpiece 106 and the cochlea implant 108.

For a relatively large distance between the coils 130, 132, i.e. for a relatively large distance of the headpiece 106 and the cochlea implant 108, or, for a relatively large distance between the headpiece 106 and the patient's head, the MTEL-RSSI value will be relatively low, and it will increase when the headpiece 108 approaches the head/skin 116. When the headpiece 106 is attached at the patient's head by the magnetic coupling between the external attachment magnet device 134 and the implant attachment magnet device 136, the MTEL-RSSI value will be maximal and will correspond to the minimum distance between the headpiece 106 and the cochlea implant 108. Since this minimum distance is primarily determined by the thickness of the skin 116 between the headpiece 106 and the cochlea implant 108, the maximal MTEL-RSSI value provides for an indirect measure of the skin thickness.

Consequently, when the headpiece 106 is becoming attached to the patient's head from an unlocked starting position, the MTEL-RSSI value will first increase rapidly from a relatively low value until a constant maximum value is achieved, which is representative of the minimum distance between the headpiece 106 and the cochlear implant 108 and which is also representative of the skin thickness. A series of MTEL-RSSI values may be measured by sampling the MTEL-RSSI value in regular time intervals while the head piece 106 is being attached to the patient's head due to the magnetic coupling between the attachment magnet device 134 and 136. In other words, the headpiece 106 moves from a magnetically decoupled starting position (or "unlocked" position) above the skin 116 (or away from the head) towards the cochlear implant 108 into a magnetically attached (or "locked") end position on the skin 116 (or at the head) by the magnetic coupling forces generated between the magnet devices 134, 136. For reducing statistical errors, such measurement series may be repeated several times, so as to obtain a plurality of measurement series.

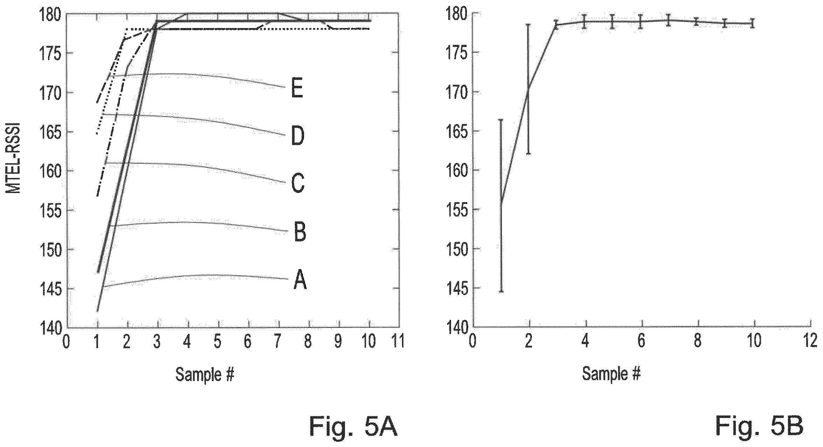

Examples of such measurement series are shown in Fig. 5A, wherein single measurement series are designated by characters "*A*" to "*E*". In the diagram of Fig. 5A the measured MTEL-RSSI value is plotted versus the sample index number (which actually corresponds to a time axis). It can be seen in Fig. 5A that for all series the behavior is similar in that the first few samples exhibit a relatively steep increase of the measured value, followed by several more or less constant values, wherein the region of the essentially constant values indicates that the headpiece 106 has reached its locked / fully attached position at the head, while the region with the increasing values indicates that the headpiece 106 is approaching the skin 116 / head.

In Fig. 5B the average of the single measurement series A to E is shown, together with error bars indicating the variance of the respective averaged MTEL-RSSI value.

Figs. 5A and 5B are representative for a system wherein the magnetic retention of the headpiece 106 by the magnetic coupling of the attachment magnet devices 134 and 136 is within the desirable range, so that the magnetic strength of the external attachment magnet device 134 matches well with the strength of the implant attachment magnet device 136. In other words, the strength of the external attachment magnet device 134 has been properly selected in the example of Figs. 5A and 5B.

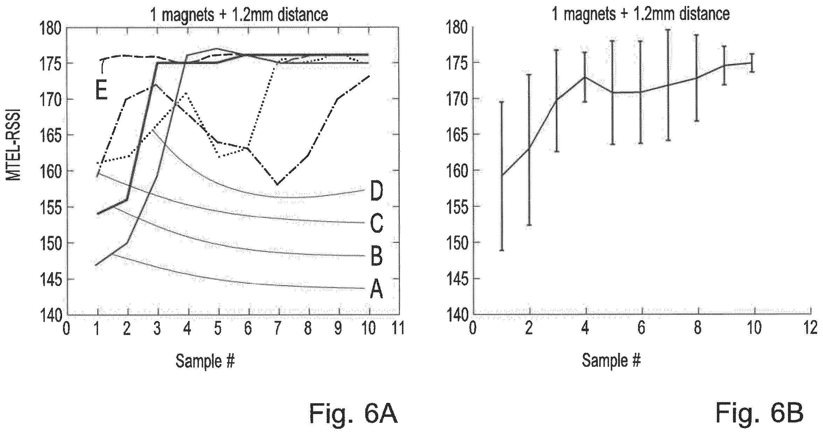

In Figs. 6A and 6B an example is shown in which the magnetic retention of the headpiece 106 is too weak, so that the magnetic attraction of the headpiece 106 actually is too low. It can be seen in Fig. 6A that for some of the measurement series attachment of the headpiece 106 was not even fully successful, since no stable attachment of the headpiece 106 could be obtained. For these series (curves C and D) no stable attachment was achieved so that the MTEL-RSSI value did not reach a constant maximum value. It can be also seen that the slope when the headpiece 106 approaches the skin 116 is lower compared to the example of Figs. 5A and 5B. Finally, it can be seen in Fig. 6B that the error bars (which are indicative of the variance) are much larger than in the case of Fig. 5B, in particular for the "full attachment range".

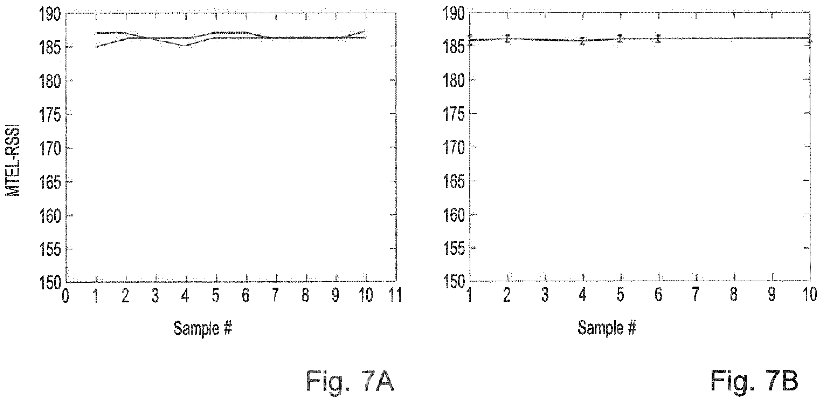

Figs. 7A and 7B illustrate an example in which the magnetic retention is much too high so that the external attachment magnet device has a much too high magnetic strength. Such high attraction force has as a result that the headpiece 106 is almost immediately locked / fully attached at the head, so that no slope is visible in Fig. 7A. Further, the variance of the MTEL-RSSI values is very low.

Measurement series of the received coil telemetry coupling strength, like the MTEL-RSSI values as shown in Figs. 5A to 7B, may be statistically analyzed to derive information concerning the magnetic coupling strength and/or the skin conditions, in particular the skin thickness. For example, an estimation of a present transcutaneous distance between the implant transceiver coil 130 and the external transceiver coil 132 and/or an estimation of a present transcutaneous magnetic coupling strength between the implant attachment magnet device 136 and the external attachment magnet device 134 may be obtained; alternatively or in addition, a target value for the desired strength of the external attachment magnet device 134 may be obtained for adjusting the transcutaneous magnetic coupling strength between the implant attachment magnet device 136 and the external attachment magnet device 134.

The analysis of the measurement series may include fitting a model function, such as an exponential function, or a response of a linear time-invariant first order system, or a parametric model to the respective series of MTEL-RSSI values, or to an averaged series of MTEL-RSSI values, so as to determine a slope and / or an amplitude of the model function. For example, the time behavior of the MTEL-RSSI values during an attachment procedure may be modelled by the function *Y*=*A**(1-exp(-*t*/*τ*)), from which the slope *τ* and the amplitude *A,* as well as residuum parameters, may be extracted.

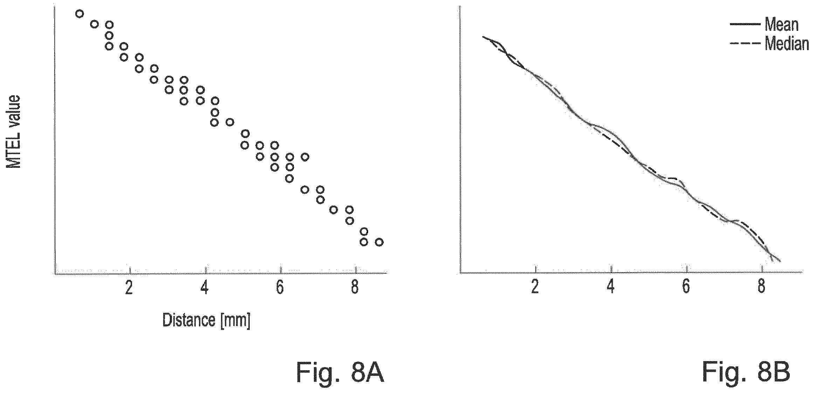

An estimation of the present transcutaneous distance between the implant transceiver coil 132 and the external transceiver coil 130 can be provided by using a predetermined relationship between the coil coupling strength values, e.g. the MTEL-RSSI values, and the distance between the implant transceiver coil 132 and the external transceiver coil 130. An example of such relationship is shown in Figs. 8A and 8B, wherein the measured MTEL-RSSI values are shown as a function of the distance in mm between the external transceiver coil 130 and the implant transceiver coil 132. Such relationship can be obtained by measuring a series of coil coupling strength values (e.g. MTEL-RSSI values) while the external component, such as the headpiece 106, with the external transceiver coil 130, is subsequently located in various positions for which the actual distance between the external transceiver coil 130 and the implant transceiver coil 132 is known. A correlation is determined between the measured coil coupling strength values and the respective known actual distances, so as to empirically obtain the relationship. In the example of Fig. 8A a plurality of measurement was conducted for some of the known distance values, so that there is some variance of the MTEL-RSSI values, whereas in Fig. 8B the values of Fig. 8A have been averaged as a mean value or as a median value, respectively. It can be seen from Fig. 8B that the relationship between the measured MTEL-RSSI value and the distance is substantially linear.

In some implementations, the relationship/correlation between the MTEL-RSSI values and the distance between the coils 130 and 132 may be implemented in a look-up table.

According to one example, an estimation of the present transcutaneous distance between the coils 130 and 132 may be obtained from the amplitude determined from the fitting of the model function (in the example mentioned above, this would be the amplitude parameter A). According to another example, an estimation of the present transcutaneous distance may be obtained from an average of those coil coupling strength values (MTEL-RSSI values) of the measured series which are constant within a given range (these are the values in the "flat" region of the curves).

As already mentioned above, an estimate of a thickness of the skin 116 located between the implant 108 and the external component (headpiece 106) may be obtained from an estimation of the present transcutaneous distance between the implant transceiver coil 132 and the external transceiver coil 130. In this regard it is to be noted that the distance between the respective coil and the housing surface which contacts the skin is generally known, so that it is fair to assume that it is substantially the skin what is located between the respective housing surfaces.

An estimation of the present transcutaneous magnetic coupling strength between the implant attachment magnet device 136 and the external attachment magnet device 134 may be obtained by analyzing the slope of the coil coupling strength values (MTEL-RSSI values) in the measured series, the variance of the coil coupling strength values in the measured series and/or the amplitude of the coil coupling strength values in the measured series. For example, as already mentioned above, a very steep slope indicates a high magnetic coupling strength, whereas a relatively shallow slope indicates a relatively low magnetic coupling strength, and a high variance of the coil coupling strength values indicates a relatively low magnetic coupling strength, whereas a low variance indicates a relatively high magnetic coupling strength. Also, the estimated skin thickness (or the estimated distance between the coils 130 and 132) may provide for an estimation of the magnetic coupling strength, since a high magnetic coupling strength results in a compression of the skin, so that the estimated skin thickness / coil distance will be lower than for a relatively weak magnetic coupling.

In some implementations, the external component, such as the headpiece 106, may comprise a unit for adjusting the magnetic coupling contribution of the external attachment magnet device 130 at a given transcutaneous distance between the external component and the implant 108. This may be implemented, for example, by making the external attachment magnet device 130 movable within the external component, so as to adjust the transcutaneous distance between the external attachment magnet device 130 and the implant attachment magnet device 132. In other words, the external attachment magnet device may be moved back and forth so as to increase or decrease the distance between the magnet devices 130, 132, thereby decreasing or increasing the magnetic coupling strength. As an alternative, the external attachment magnet device 130 may comprise at least one electropermanent magnet which can be electrically activated or deactivated so as to increase or decrease the magnetic coupling strength.

In some implementations, such magnetic coupling adjustment unit may be controlled by a target value of the magnetic coupling derived from an estimation of the magnetic coupling strength obtained from analyzing MTEL-RSSI measurement series.

The above-mentioned coil coupling strength measurement control functionality and analyzing functionality may be implemented in an analyzing unit which is physically implemented in a device which is separate from the external component (headpiece 106) and which is communicatively coupled to the external component. According to one example, the analyzing unit may be implemented in a fitting device of an audiologist. In other examples, the analyzing unit may be implemented in a mobile phone or a tablet computer of the recipient.

In the example illustrated in Fig. 1, an analyzing unit 190 is implemented in a device 192 which comprises a wireless interface 194 for wireless communication with the sound processor 104, such as a Bluetooth interface. The device 192 in addition may comprise a memory 196 and an output unit 198. The output unit 198 may be used for notifying or warning a user, such as the recipient. Alternatively or in addition, the output unit 198 may comprise an interface with other devices or with a network, such as a cloud 199.

When the analyzing unit 190 is implemented in a fitting device of the audiologist, the analyzing unit 190 may provide for an estimation of the present transcutaneous magnetic coupling strength or for a recommended target value for the transcutaneous coupling strength, so as to assist the audiologist in selecting the optimal external attachment magnet device strength. To this end, a look-up table may be used for finding the target value, i.e., to find the optimal magnetic coupling strength.

In implementations in which the analyzing unit 190 is implemented in a personal electronic device of the patient, such as in a smartphone, the patient may be provided with notifications or warnings via the output unit 198, which may include a display and/or a speaker for presenting visual information, like text messages and optical signals, and/or acoustic information, like audio messages or alert sounds, to the patient.

In some implementations, a thickness of the skin 116 located between the implant 108 and the headpiece 106 may be monitored by regularly repeating an estimation of the thickness of the skin 116 from an estimation of the present transcutaneous distance between the coils 130 and 132. In other words, the measurements necessary for estimating the thickness of the skin 116 may be regularly repeated, so that the measuring of series of coil coupling strength values may be regularly repeated by the measurement unit 170. For example, a series of coil coupling strength values may be measured on a daily basis when the headpiece 106, after the sound processor 104 has been turned on, is becoming attached to the head by the patient. The estimation of the thickness of the skin 116, which is regularly obtained from such daily measurements, may be recorded as a function of time and/or as a histogram so as to identify critical changes in the estimated skin thickness. A corresponding message or warning then may be provided via the output unit 198 to the patient or, via a network / cloud, to the implantation clinic or the audiologist of the patient.

In some implementations, an inflammation warning may be output when an increase in the estimated skin thickness is found to exceed a predetermined inflammation threshold. Further, a skin-breakdown warning may be output when a decrease in the estimated skin thickness is found to exceed a predetermined skin-breakdown threshold, indicating that the skin thickness becomes too low.

In some implementations, in the decision of whether to output an inflammation warning or a skin-breakdown warning or not, not only the absolute value of the estimated skin thickness but also the rate of change of the estimated skin thickness may be taken into account. For example, an inflammation warning may be output only in case that the estimated skin thickness is found to exceed a predetermined inflammation threshold and that the rate at which the estimated skin thickness increases is found to be above a predetermined rate threshold (which condition is an indication that the estimated skin thickness increases relatively fast). This may be helpful for distinguishing a relatively fast inflammation induced increase of the estimated skin thickness from a very slow increase of the estimated skin thickness due to hair growing.

When an inflammation or skin irritation occurs, the skin will become thicker, so that the distance between the coils 130 and 132 becomes larger, which, in turn, results in lower measured MTEL-RSSI values and hence in an increase in the estimated skin thickness. In such case, an alert signal should be given to the patient so as to counsel with the clinic.

Shortly after implantation of the cochlear implant, a decrease of the estimated skin thickness, which may be detected from the resulting increase of the MTEL-RSSI values, may indicate that the swelling caused by the surgery decreases as part of the recovery process. However, a decrease of the estimated skin thickness over a longer time period may indicate that actually the skin under the headpiece 106 becomes thinner due to an overly strong magnetic coupling between the headpiece 106 and the implant 108, which in the worst case may result in skin breakdown. To avoid such skin breakdown, an alert signal should be given that the patient should counsel the clinic, as in case of a detection of an inflammation. The measurement data and/or the estimations resulting from the measurement data may be stored in the memory 196 of the device 192 and/or it may be sent to the cloud 199 via the output unit 198.

Also the magnetic coupling strength as such may be monitored by the device 192. For example, a warning may be output to use a weaker external attachment magnet device 134 when the slope of the measured coil coupling strength values is found to be above a high slope threshold and/or when the variation of the variance of the measured coil coupling strength values is found to be below a low variance threshold. As already discussed above with regard to Figs. 7A and 7B, a too strong external attachment magnet device 130 may result in MTEL-RSSI values which show no or only little variation and which have no or a very steep slope.

On the other hand, a recommendation to use a stronger external attachment magnet device 130 may be output when the slope of the measured coil coupling strength values is below a low slope threshold and/or when the variance of the measured coil coupling strength values is above a high variance threshold. As already discussed above with regard to Fig. 6A and 6B, a too weak external attachment magnet device 130 may result in a high variability of the measured MTEL-RSSI values and a relatively shallow slope.

As already mentioned above, the device 192 may provide for a recommendation of the optimal strength of the external attachment magnet device 130. Since the optimal strength depends on the skin thickness and since the amplitude of the measured MTEL-RSSI values (which is the asymptotic maximum value) likewise is correlated with the skin thickness, the optimal magnetic strength may be recommended based on a skin thickness estimation. However, since it is per se not known whether such recommendation, which may be initially given to the audiologist or the patient, is actually followed by the patient, it is helpful to monitor the magnetic strength during the actual use of the cochlea implant system by the patient.

Measurements of coil coupling strength values can be utilized not only for estimations of the transcutaneous distance between the implant transceiver coil and the external transceiver coil and / or for estimations of the transcutaneous magnetic coupling strength between the implant attachment magnet device and the external attachment magnet device, as described so far, but alternatively or in addition such measurements also may be used for judging whether or not there was an temporary interruption in the supply of the external transceiver coil with signals to be transmitted by the external transceiver coil, so as to detect hardware defects, such as broken cables or loose contacts, in the signal supply chain to the external transceiver coil.

Generally, hardware defects in the signal supply chain to the external transceiver coil, such a broken cables or loose contacts, will result in audible stops and restarts of the stimulation pattern applied by the cochlear implant. Children or poor performers (patients with low hearing capability) probably may not hear such interruptions in the stimulation. Good performers (patients with good hearing capability) will perceive such interruptions, but still may be unsure what they can do to fix this issue. Therefore, the detection of hardware defects in the signal supply chain to the external transceiver coil is desirable.

In the example shown in Figs. 2 and 3 the processed signals generated in the BTE unit 175 are sent through a cable connection 177, which may be detachable, to the headpiece 106 with the external transceiver coil 130 for transcutaneous transmission to the implant coil 132. Potential hardware defects in the supply chain of the processed signals to the external transceiver coil 130 are broken wires in the cable connection or electrical contact problems of the cable connection 177 at the detachable contact points with the headpiece 106 and/or the BTE unit 175.

During normal use of the cochlear implant system, the headpiece 106 is worn at the head and remains inductively coupled, via the external transceiver coil 130 and the implant transceiver coil 132, to the cochlear implant 108, so that the sound processor 104 remains connected with the cochlear implant 104. If there is a temporary interruption of the electrical connection in the signal supply chain to external transceiver coil 130, for example due to a loose contact in the cable connection 177, the transcutaneous inductive coupling will be lost and the cochlea stimulation via the cochlear implant 108 will stop, so that the sound processor 104 needs to connect again to the cochlear implant 108 via inductive coupling of the coils 130 and 132 when the electrical connection in the signal supply chain to external transceiver coil 130 is restored.

Since the headpiece 106 is stationary relative to the cochlear implant 108 when the inductive coupling is resumed, measuring a series of MTEL-RSSI values once the inductive coupling is restored will result in a series with very little variation of the MTEL-RSSI values, so that a very flat distribution of values with very low variance will be obtained. The reason is that, since the headpiece is not approaching from a distance towards the head (which would result in low initial MTEL-RSSI values which then increase while the headpiece approaches the head until a stable plateau is reached when the headpiece is fixed at the head) there will be from the first sample on high stable MTEL-RSSI values.

Actually, the shape of the curve of the measured MTEL-RSSI values will be similar to that resulting from cases in which there is a very strong magnetic coupling between the headpiece and the cochlear implant, when the headpiece approaches the head, as illustrated in Figs. 7A and 7B.

Thus, the occurrence of temporary interruptions of the supply of the external transceiver coil with signals to be transmitted - and hence the presence of hardware defects in the signal supply chain - may be detected when it is found (i) that all measured coil coupling strength values are within a given range or (ii) that a slope of the coil coupling strength values in the measured series of coil coupling strength values is within a given range around zero or (iii) that the variance of the coil coupling strength values in the measured series of coil coupling strength values is below a given threshold.

When an occurrence of temporary interruptions of the signal supply of the external transceiver coil has been detected by the analyzing unit 190, a corresponding warning notification may be output to the patient, such as by a warning audio message or a warning sound signal like a "beep" generated by the sound processor 104 and presented via the cochlear implant 108, so that patient can take care of fixing the underlying hardware defect.

The phrases "in some implementations," "according to some implementations," "in the implementations shown," "in other implementations," and generally mean the particular feature, structure, or characteristic following the phrase is included in at least one implementation of the disclosure, and may be included in more than one implementation. In addition, such phrases do not necessarily refer to the same embodiments or different implementations.

The above detailed description of examples of the disclosure is not intended to be exhaustive or to limit the disclosure to the precise form disclosed above. While specific examples for the disclosure are described above for illustrative purposes, various equivalent modifications are possible within the scope of the disclosure, as those skilled in the relevant art will recognize. For example, while processes or blocks are presented in a given order, alternative implementations may perform routines having steps, or employ systems having blocks, in a different order, and some processes or blocks may be deleted, moved, added, subdivided, combined, and/or modified to provide alternative or subcombinations. Each of these processes or blocks may be implemented in a variety of different ways. Also, while processes or blocks are at times shown as being performed in series, these processes or blocks may instead be performed or implemented in parallel, or may be performed at different times. Further any specific numbers noted herein are only examples: alternative implementations may employ differing values or ranges.

## Claims

1. A hearing prosthesis system comprising
a hearing implant (108) implantable within a patient and including an implant transceiver coil (132) and an implant attachment magnet device (136);
a measurement unit (170);
an external component (106) including an external transceiver coil (134) and an external attachment magnet device (130), wherein the external component comprises or is connected to the measurement unit (170) for measuring coil coupling strength values indicative of the received coil telemetry coupling strength ("MTEL-RSSI") when the external transceiver coil and the implant transceiver coil are inductively coupled; and
an analyzing unit (190) configured to control the measurement unit, to receive coil coupling strength values measured by the measurement unit and to analyze the received coil coupling strength values so as to output at least one of
an estimation of a present transcutaneous distance between the implant transceiver coil and the external transceiver coil,
an estimation of a present transcutaneous magnetic coupling strength between the implant attachment magnet device and the external attachment magnet device, and
a target value of the transcutaneous magnetic coupling strength for adjusting the transcutaneous magnetic coupling strength between the implant attachment magnet device and the external attachment magnet device.

2. The system of claim 1, wherein the analyzing unit (190) is configured to cause the measurement unit (170) to measure a series of coil coupling strength values while the external component (106) moves from a magnetically decoupled starting position above the patient's skin (116) towards the hearing implant (108) into a magnetically attached end position on the skin by magnetic coupling forces between the implant attachment magnet device (136) and the external attachment magnet device (134).

3. The system of claim 2, wherein the analyzing unit (190) is configured to cause the measurement unit (170) to repeatedly measure a series of coil coupling strength values while the external component (106) moves from a magnetically decoupled starting position above the skin towards the hearing implant into a magnetically attached end position on the skin by magnetic coupling forces between the implant attachment magnet device (136) and the external attachment magnet device (134), so as to obtain a plurality of series of coil coupling strength values.

4. The system of claim 3, wherein the analyzing unit (190) is configured to conduct a statistical analysis, in particular including an averaging procedure, over the measured series of coil coupling strength values or over the measured plurality of series of coil coupling strength values, respectively.

5. The system of one of claims 3 and 4, wherein the analyzing unit is configured to fit a model function or a parametric model to the measured series of coil coupling strength values or to an averaged series of coil coupling strength values, so as to determine a slope and / or an amplitude of the model function or parametric model.

6. The system of one of the preceding claims, wherein the analyzing unit (190) is configured to provide an estimation of the present transcutaneous distance between the implant transceiver coil (132) and the external transceiver coil (130) by using a predetermined relationship between the coil coupling strength values and the distance between the implant transceiver coil and the external transceiver coil.

7. The system of claim 5, wherein the analyzing unit (190) is configured to provide an estimation of the present transcutaneous distance between the implant transceiver coil (132) and the external transceiver coil (130) from the amplitude determined from the fitting of the model function or parametric model.

8. The system of one of claims 6 and 7, wherein the analyzing unit (190) is configured to estimate a thickness of the skin (116) located between the hearing implant (108) and the external component (106) from the estimation of the present transcutaneous distance between the implant transceiver coil (132) and the external transceiver coil (130).

9. The system of one of claims 2 and 3, wherein the analyzing unit (190) is configured to provide for an estimation of a transcutaneous magnetic coupling strength between the implant attachment magnet device (136) and the external attachment magnet device (134) by analyzing the measured series of coil coupling strength values, or the measured plurality of series of coil coupling strength values, respectively.

10. The system of one of the preceding claims, wherein the external component (106) comprises a unit for adjusting the magnetic coupling strength contribution of the external attachment magnet device (134), and wherein the unit for adjusting the magnetic coupling strength contribution of the external attachment magnet device (134) is configured to activate or deactivate one or more electropermanent magnets or to move the external attachment magnet device within the external component (106) so as to adjust the transcutaneous distance between the external attachment magnet device and the implant attachment magnet device (136).

11. The system of one of the preceding claims, wherein the analyzing unit (190) is implemented in a device (192) which is separate from the external component (106) and which configured to be communicatively coupled to the external component, wherein the analyzing unit (190) is implemented in a fitting device, a mobile phone, a tablet computer, or a computing cloud.

12. The system of claim 8, wherein the analyzing unit (190) is configured to monitor a thickness of the skin located between the hearing implant (108) and the external component (106) over time by regularly repeating an estimation of thickness of the skin from an estimation of the present transcutaneous distance between the implant transceiver coil (132) and the external transceiver coil (130).

13. The system of claim 12, wherein the analyzing unit (190) is configured to have the estimation of the thickness of the skin (116) recorded as a function of time, and wherein the analyzing unit (190) is configured to output at least one of: (1) an inflammation warning when an increase in the estimation of the skin thickness exceeds a predetermined inflammation threshold, (2) a skin-breakdown warning when a decrease in the estimation of the skin thickness exceeds a predetermined breakdown threshold, and (3) an inflammation warning or a skin-breakdown warning, respectively, when the rate of change in the estimation of the skin thickness exceeds a predetermined rate threshold.

14. The system of claim 9, wherein the analyzing unit (190) is configured to provide for an estimation of a transcutaneous magnetic coupling strength between the implant attachment magnet device (136) and the external attachment magnet device (134) by analyzing at least one of (i) the slope of the coil coupling strength values in the measured series of coil coupling strength values or in the measured plurality of series of coil coupling strength values, (ii) the variance of the coil coupling strength values in the measured series of coil coupling strength values or in the measured plurality of series of coil coupling strength values, and (iii) the amplitude of the coil coupling strength values in the measured series of coil coupling strength values or in the measured plurality of series of coil coupling strength values, and wherein the analyzing unit (190) is configured to output at least one of: (1) a warning to use a weaker external attachment magnet device (134) when the steepness of the slope of the measured coil coupling strength values in the measured series or in the measured plurality of series of coil coupling strength values is above a high slope threshold and/or when the variation of the variance of the coil coupling strength values in the measured series of coil coupling strength values or in the measured plurality of series of coil coupling strength values is below a low variance threshold, (2) a recommendation to use a stronger external attachment magnet device (134) when the steepness of the slope of the coil coupling strength values in the measured series of coil coupling strength values or in the measured plurality of series of coil coupling strength values is below a low slope threshold and/or when the variation of the variance of the coil coupling strength values in the measured series of coil coupling strength values or in the measured plurality of series of coil coupling strength values is above a high variance threshold, and (3) a recommendation for an optimal strength of the external attachment magnet device (134).

15. The system of one of the preceding claims, wherein the hearing implant is a cochlear implant (108), a middle-ear implant, an auditory brain stem implant, or a bone conduction implant, wherein the external component is a headpiece (106) for transmitting stimulation signals and power via the external transceiver coil (130) to the implant transceiver coil (132), and wherein the external component (106) is connected to a sound processor (104) configured to generate stimulation signals.

## Patentansprüche

1. Hörprothesensystem mit:
einem Hörimplantat (108), welches in einem Patienten implantierbar ist und eine Implantat-Sende-Empfänger-Spule (132) sowie eine Implantat-Befestigungsmagnetvorrichtung (136) aufweist;
einer Messeinheit (170);
einer externen Komponente (106) mit einer externen Sende-Empfänger-Spule (134) sowie einer externen Befestigungsmagnetvorrichtung (130), wobei die externe Komponente die Messvorrichtung (170) beinhaltet oder mit dieser verbunden ist, um Spulenkupplungsstärkenwerte zu messen, die bezeichnend für die empfangene Spulentelemetrie-Kopplungsstärke ("MTEL-RSSI") sind, wenn die externe Sende-Empfänger-Spule und die Implantat-Sende-Empfänger-Spule induktiv gekoppelt sind; und
eine Analyseeinheit (190), die ausgebildet ist, um die Messeinheit zu steuern, von der Messeinheit gemessene Spulenkopplungsstärkewerte zu empfangen und die empfangenen Spulenkopplungsstärkewerte zu analysieren, um mindestens eine der folgenden Größen auszugeben:
eine Abschätzung eines momentanen transkutanen Abstands zwischen der Implantat-Sende-Empfänger-Spule und der externen Sende-Empfänger-Spule,
eine Abschätzung einer momentanen transkutanen magnetischen Kopplungsstärke zwischen der Implantat-Befestigungsmagnetvorrichtung und der externen Befestigungsmagnetvorrichtung, und
einen Zielwert der transkutanen magnetischen Kopplungsstärke zum Einstellen der transkutanen magnetischen Kopplungsstärke zwischen der Implantat-Befestigungsmagnetvorrichtung und der externen Befestigungsmagnetvorrichtung.

2. System gemäß Anspruch 1, wobei die Analyseeinheit (190) ausgebildet ist, um die Messeinheit (170) zu veranlassen, eine Reihe von Spulenkopplungsstärkenwerten zu messen, während sich die externe Komponente (106) mittels magnetischer Kopplungskräfte zwischen der Implantat-Befestigungsmagnetvorrichtung (136) und der externen Befestigungsmagnetvorrichtung (134) von einer magnetisch entkoppelten Startposition über der Haut (116) des Patienten zu dem Hörimplantat (108) hin in eine magnetisch befestigte Endposition auf der Haut bewegt.

3. System gemäß Anspruch 2, wobei die Analyseeinheit (190) ausgebildet ist, um die Messeinheit (170) zu veranlassen, eine Reihe von Spulenkopplungsstärkenwerten wiederholt zu messen, während sich die externe Komponente mittels magnetischer Kopplungskräfte zwischen der Implantat-Befestigungsmagnetvorrichtung (136) und der externen Befestigungsmagnetvorrichtung (134) von einer magnetisch entkoppelten Startposition über der Haut zu dem Hörimplantat hin in eine magnetisch befestigte Endposition auf der Haut bewegt, um eine Mehrzahl von Reihen von Spulenkopplungsstärkenwerten zu erhalten.

4. System gemäß Anspruch 3, wobei die Analyseeinheit (190) ausgebildet ist, um eine statistische Analyse, insbesondere einschließlich einer Ermittlungsprozedur, bezüglich der gemessenen Reihe von Spulenkopplungsstärkenwerten bzw. bezüglich der gemessenen Mehrzahl von Reihen von Spulenkopplungsstärkenwerten ausführen.

5. System gemäß einem der Ansprüche 3 oder 4, wobei die Analyseeinheit ausgebildet ist, um eine Modellfunktion oder ein parametrisches Modell an die gemessenen Reihen von Spulenkopplungsstärkenwerten oder an eine gemittelte Reihe von Spulenkopplungsstärkenwerten anzupassen, um eine Steigung und/oder eine Amplitude der Modellfunktion oder des parametrischen Modells zu bestimmen.

6. System gemäß einem der Ansprüche, wobei die Analyseeinheit (190) ausgebildet ist, um eine Abschätzung des momentanen transkutanen Abstands zwischen der Implantat-Sende-Empfänger-Spule (132) und der externen Sende-Empfänger-Spule (130) zu liefern, indem eine vorbestimmte Beziehung zwischen den Spulenkopplungsstärkenwerten und dem Abstand zwischen der Implantat-Sende-Empfänger-Spule und der externen Sende-Empfänger-Spule verwendet wird.

7. System gemäß Anspruch 5, wobei die Analyseeinheit (190) ausgebildet ist, um eine Abschätzung des momentanen transkutanen Abstands zwischen der Implantat-Sende-Empfänger-Spule (132) und der externen Sende-Empfänger-Spule (130) aus der aus der Anpassung der Modellfunktion oder des parametrischen Modells bestimmten Amplitude zu liefern.

8. System gemäß einem der Ansprüche 6 oder 7, wobei die Analyseeinheit (190) ausgebildet ist, um eine Dicke der zwischen dem Hörimplantat (108) und der externen Komponente (106) angeordneten Haut (116) aus der Abschätzung des momentanen transkutanen Abstands zwischen der Implantat-Sende-Empfänger-Spule (132) und der externen Sende-Empfänger-Spule (130) abzuschätzen.

9. System gemäß einem der Ansprüche 2 oder 3, wobei die Analyseeinheit (190) ausgebildet ist, um eine Abschätzung einer transkutanen magnetischen Kopplungsstärke zwischen der Implantat-Befestigungsmagnetvorrichtung (136) und der externen Befestigungsmagnetvorrichtung (134) zu liefern, indem die gemessene Reihe von Spulenkopplungsstärkenwerten bzw. die gemessene Mehrzahl von Reihen von Spulenkopplungsstärkenwerten analysiert werden.

10. System gemäß einem der vorhergehenden Ansprüche, wobei die externe Komponente (106) eine Einheit zum Einstellen des Magnetkopplungsstärkenbeitrags der externen Befestigungsmagnetvorrichtung (134) aufweist, und wobei die Einheit zum Einstellen des Magnetkopplungsstärkenbeitrags der externen Befestigungsmagnetvorrichtung (134) ausgebildet ist, um mindestens einen Elektropermanentmagneten zu aktivieren oder zu deaktivieren oder die externe Befestigungsmagnetvorrichtung innerhalb der externen Komponente (106) zu bewegen, um den transkutanen Abstand zwischen der externen Befestigungsmagnetvorrichtung und der Implantat-Befestigungsmagnetvorrichtung (136) einzustellen.

11. System gemäß einem der vorhergehenden Ansprüche, wobei die Analyseeinheit (190) in einer Vorrichtung (192) implementiert ist, welche separat von der externen Komponente (106) ausgebildet ist und welche ausgebildet ist, um kommunikativ mit der externen Komponente gekoppelt zu sein, wobei die Analyseeinheit (190) in einer Fitting-Vorrichtung, einem Mobiltelefon, einem Tabletcomputer oder einer Computercloud implementiert ist.

12. System gemäß Anspruch 8, wobei die Analyseeinheit (190) ausgebildet ist, um eine Dicke der zwischen dem Hörimplantat (108) und der externen Komponente (106) angeordneten Haut über die Zeit zu überwachen, indem regelmäßig eine Abschätzung der Dicke der Haut aus einer Abschätzung des momentanen transkutanen Abstands zwischen der Implantat-Sende-Empfänger-Spule (132) und der externen Sende-Empfänger-Spule (130) wiederholt wird.

13. System gemäß Anspruch 12, wobei die Analyseeinheit (190) ausgebildet ist, um die Abschätzung der Dicke der Haut (116) als Funktion der Zeit aufzuzeichnen, und wobei die Analyseeinheit (190) ausgebildet ist, um (1) eine Entzündungswarnung auszugeben, wenn ein Anstieg der Abschätzung der Hautdicke eine vorbestimmte Entzündungsschwelle übersteigt, (2) eine Hautzusammenbruchwarnung auszugeben, wenn eine Abnahme der Abschätzung der Hautdicke eine vorbestimmte Zusammenbruchschwelle übersteigt, und/oder (3) eine Entzündungswarnung bzw. eine Hautzusammenbruchwarnung auszugeben, wenn die Änderungsrate der Abschätzung der Hautdicke eine vorbestimmte Ratenschwelle übersteigt.

14. System gemäß Anspruch 9, wobei die Analyseeinheit (190) ausgebildet ist, um eine Abschätzung einer transkutanen Magnetkopplungsstärke zwischen der Implantat-Befestigungsmagnetvorrichtung und der externen Befestigungsmagnetvorrichtung (134) zu liefern, indem mindestens eine der folgenden Größen realisiert wird:
(i) die Steigung der Spulenkopplungsstärkenwerte in der gemessenen Reihe von Spulenkopplungsstärkenwerten oder in der gemessenen Mehrzahl von Reihen von Spulenkopplungsstärkenwerten, (ii) die Varianz der Spulenkopplungsstärkenwerte in der gemessenen Reihe von Spulenkopplungsstärkenwerten oder in der gemessenen Mehrzahl von Reihen von Spulenkopplungsstärkenwerten, und (iii) die Amplitude der Spulenkopplungsstärkenwerte in der gemessenen Reihe von Spulenkopplungsstärkenwerten oder in der gemessenen Mehrzahl von Reihen von Spulenkopplungsstärkenwerten, und wobei die Analyseeinheit (190) ausgebildet ist, um mindestens Folgendes auszugeben: (1) eine Warnung, eine schwächere externe Befestigungsmagnetvorrichtung (134) zu verwenden, wenn die Steilheit der Steigung der gemessenen Spulenkopplungsstärkenwerte in der gemessenen Reihe oder in der gemessenen Mehrzahl von Reihen von Spulenkopplungsstärkenwerten oberhalb einer Hochsteilheitsschwelle liegt und/oder wenn die Variation der Varianz der Spulenkopplungsstärkenwerte in der gemessenen Reihe von Spulenkopplungsstärkenwerten oder in der gemessenen Mehrzahl von Reihen von Spulenkopplungsstärkenwerten unterhalb einer Niedrigvarianzschwelle liegt; (2) eine Empfehlung, eine stärkere externe Befestigungsmagnetvorrichtung (134) zu verwenden, wenn die Steilheit der Steigung der Spulenkopplungsstärkenwerte in der gemessenen Reihe von Spulenkopplungsstärkenwerten oder in der gemessenen Mehrzahl von Reihen von Spulenkopplungsstärkenwerten unterhalb einer Niedrigsteigungsschwelle liegt und/oder wenn die Variation der Varianz der Spulenkopplungsstärkenwerte in der gemessenen Reihe von Spulenkopplungsstärkenwerten oder in der gemessenen Mehrzahl von Reihen von Spulenkopplungsstärkenwerten oberhalb einer Hochvarianzschwelle liegt; und/oder (3) eine Empfehlung für eine optimale Stärke der externen Befestigungsmagnetvorrichtung (134).

15. System gemäß einem der vorhergehenden Ansprüche, wobei es sich bei dem Hörimplantat um ein Cochleaimplantat (108), ein Mittelohrimplantat, ein Hörhirnstammimplantat oder ein Knochenleitungsimplantat handelt, wobei es sich bei der externen Komponente um ein Kopfstück (106) zum Senden von Stimulationssignalen und Energie über die externe Sende-Empfänger-Spule (130) zu der Implantat-Sende-Empfänger-Spule (132) handelt, und wobei die externe Komponente (106) mit einem Schallprozessor (104) verbunden ist, um Stimulationssignale zu erzeugen.

## Revendications

1. Système de prothèse auditive comprenant
un implant auditif (108) implantable à l'intérieur d'un patient et comprenant une bobine émettrice-réceptrice d'implant (132) et un dispositif magnétique de fixation d'implant (136) ;
une unité de mesure (170);
un composant externe (106) comprenant une bobine émettrice-réceptrice externe (134) et un dispositif magnétique de fixation externe (130), le composant externe comprenant ou étant connecté à l'unité de mesure (170) pour mesurer des valeurs de force de couplage de bobine indicatives de la force de couplage télémétrique de bobine reçue (« MTEL-RSSI ») lorsque la bobine émettrice-réceptrice externe et la bobine émettrice-réceptrice d'implant sont couplées par induction ; et
une unité d'analyse (190) configurée pour commander l'unité de mesure, pour recevoir des valeurs de force de couplage de bobine mesurées par l'unité de mesure et pour analyser les valeurs de force de couplage de bobine reçues de manière à produire au moins une des valeurs parmi
une estimation d'une distance transcutanée présente entre la bobine émettrice-réceptrice d'implant et la bobine émettrice-réceptrice externe,
une estimation d'une force de couplage magnétique transcutanée présente entre le dispositif magnétique de fixation d'implant et le dispositif magnétique de fixation externe, et
une valeur cible de la force de couplage magnétique transcutanée pour ajuster la force de couplage magnétique transcutanée entre le dispositif magnétique de fixation d'implant et le dispositif magnétique de fixation externe.

2. Système selon la revendication 1, l'unité d'analyse (190) étant configurée pour amener l'unité de mesure (170) à mesurer une série de valeurs de force de couplage de bobine tandis que le composant externe (106) se déplace d'une position de départ découplée magnétiquement au-dessus de la peau du patient (116) vers l'implant auditif (108) dans une position d'extrémité fixée magnétiquement sur la peau par des forces de couplage magnétiques entre le dispositif magnétique de fixation d'implant (136) et le dispositif magnétique de fixation externe (134).

3. Système selon la revendication 2, l'unité d'analyse (190) étant configurée pour amener l'unité de mesure (170) à mesurer de manière répétée une série de valeurs de force de couplage de bobine tandis que le composant externe (106) se déplace d'une position de départ découplée magnétiquement au-dessus de la peau vers l'implant auditif dans une position d'extrémité fixée magnétiquement sur la peau par des forces de couplage magnétiques entre le dispositif magnétique de fixation d'implant (136) et le dispositif magnétique de fixation externe (134), de manière à obtenir une pluralité de séries de valeurs de force de couplage de bobine.

4. Système selon la revendication 3, l'unité d'analyse (190) étant configurée pour effectuer une analyse statistique, incluant en particulier une procédure de moyennage, sur la série mesurée de valeurs de force de couplage de bobine ou sur la pluralité mesurée de séries de valeurs de force de couplage de bobine, respectivement.

5. Système selon l'une des revendications 3 et 4, l'unité d'analyse étant configurée pour ajuster une fonction de modèle ou un modèle paramétrique à la série mesurée de valeurs de force de couplage de bobine ou à une série moyennée de valeurs de force de couplage de bobine, de manière à déterminer une pente et/ou une amplitude de la fonction de modèle ou du modèle paramétrique.

6. Système selon l'une des revendications précédentes, l'unité d'analyse (190) étant configurée pour fournir une estimation de la distance transcutanée présente entre la bobine émettrice-réceptrice d'implant (132) et la bobine émettrice-réceptrice externe (130) en utilisant une relation prédéterminée entre les valeurs de force de couplage de bobine et la distance entre la bobine émettrice-réceptrice d'implant et la bobine émettrice-réceptrice externe.

7. Système selon la revendication 5, l'unité d'analyse (190) étant configurée pour fournir une estimation de la distance transcutanée présente entre la bobine émettrice-réceptrice d'implant (132) et la bobine émettrice-réceptrice externe (130) à partir de l'amplitude déterminée à partir de l'ajustement de la fonction de modèle ou du modèle paramétrique.

8. Système selon l'une des revendications 6 et 7, l'unité d'analyse (190) étant configurée pour estimer une épaisseur de la peau (116) située entre l'implant auditif (108) et le composant externe (106) à partir de l'estimation de la distance transcutanée présente entre la bobine émettrice-réceptrice d'implant (132) et la bobine émettrice-réceptrice externe (130).

9. Système selon l'une des revendications 2 et 3, l'unité d'analyse (190) étant configurée pour fournir une estimation d'une force de couplage magnétique transcutanée entre le dispositif magnétique de fixation d'implant (136) et le dispositif magnétique de fixation externe (134) en analysant la série mesurée de valeurs de force de couplage de bobine, ou la pluralité mesurée de séries de valeurs de force de couplage de bobine, respectivement.

10. Système selon l'une des revendications précédentes, le composant externe (106) comprenant une unité pour ajuster la contribution de force de couplage magnétique du dispositif magnétique de fixation externe (134), et l'unité pour ajuster la contribution de force de couplage magnétique du dispositif magnétique de fixation externe (134) étant configurée pour activer ou désactiver un ou plusieurs aimants électropermanents ou pour déplacer le dispositif magnétique de fixation externe à l'intérieur du composant externe (106) de manière à ajuster la distance transcutanée entre le dispositif magnétique de fixation externe et le dispositif magnétique de fixation d'implant (136).

11. Système selon l'une des revendications précédentes, l'unité d'analyse (190) étant mise en œuvre dans un dispositif (192) qui est séparé du composant externe (106) et qui est configuré pour être couplé de manière communicative au composant externe, l'unité d'analyse (190) étant mise en œuvre dans un dispositif d'adaptation, un téléphone mobile, un ordinateur tablette ou un nuage informatique.

12. Système selon la revendication 8, l'unité d'analyse (190) étant configurée pour surveiller une épaisseur de la peau située entre l'implant auditif (108) et le composant externe (106) au cours du temps en répétant régulièrement une estimation de l'épaisseur de la peau à partir d'une estimation de la distance transcutanée présente entre la bobine émettrice-réceptrice d'implant (132) et la bobine émettrice-réceptrice externe (130).

13. Système selon la revendication 12, l'unité d'analyse (190) étant configurée pour faire enregistrer l'estimation de l'épaisseur de la peau (116) en fonction du temps, et l'unité d'analyse (190) étant configurée pour délivrer en sortie au moins l'un des éléments suivants : (1) un avertissement d'inflammation lorsqu'une augmentation de l'estimation de l'épaisseur de la peau dépasse un seuil d'inflammation prédéterminé, (2) un avertissement de rupture de la peau lorsqu'une diminution de l'estimation de l'épaisseur de la peau dépasse un seuil de rupture prédéterminé, et (3) un avertissement d'inflammation ou un avertissement de rupture de la peau, respectivement, lorsque le taux de changement dans l'estimation de l'épaisseur de la peau dépasse un seuil de taux prédéterminé.

14. Système selon la revendication 9, l'unité d'analyse (190) étant configurée pour fournir une estimation d'une force de couplage magnétique transcutanée entre le dispositif magnétique de fixation d'implant (136) et le dispositif magnétique de fixation externe (134) en analysant au moins l'une des valeurs parmi (i) la pente des valeurs de force de couplage de bobine dans la série mesurée de valeurs de force de couplage de bobine ou dans la pluralité mesurée de séries de valeurs de force de couplage de bobine, (ii) la variance des valeurs de force de couplage de bobine dans la série mesurée de valeurs de force de couplage de bobine ou dans la pluralité mesurée de séries de valeurs de force de couplage de bobine, et (iii) l'amplitude des valeurs de force de couplage de bobine dans la série mesurée de valeurs de force de couplage de bobine ou dans la pluralité mesurée de séries de valeurs de force de couplage de bobine, et l'unité d'analyse (190) étant configurée pour produire au moins l'un des éléments parmi: (1) un avertissement d'utilisation d'un dispositif magnétique de fixation externe plus faible (134) lorsque la raideur de la pente des valeurs de force de couplage de bobine mesurées dans la série mesurée ou dans la pluralité mesurée de séries de valeurs de force de couplage de bobine est supérieure à un seuil de pente élevé et/ou lorsque la variation de la variance des valeurs de force de couplage de bobine dans la série mesurée de valeurs de force de couplage de bobine ou dans la pluralité mesurée de séries de valeurs de force de couplage de bobine est inférieure à un seuil de variance faible, (2) une recommandation d'utilisation d'un dispositif magnétique de fixation externe plus fort (134) lorsque la raideur de la pente des valeurs de force de couplage de bobine dans la série mesurée de valeurs de force de couplage de bobine ou dans la pluralité mesurée de séries de valeurs de force de couplage de bobine est inférieure à un seuil de pente faible et/ou lorsque la variation de la variance des valeurs de force de couplage de bobine dans la série mesurée de valeurs de force de couplage de bobine ou dans la pluralité mesurée de séries de valeurs de force de couplage de bobine est supérieure à un seuil de variance élevé, et (3) une recommandation pour une force optimale du dispositif magnétique de fixation externe (134).

15. Système selon l'une des revendications précédentes, l'implant auditif étant un implant cochléaire (108), un implant de l'oreille moyenne, un implant auditif du tronc cérébral ou un implant de conduction osseuse, ledit composant externe étant une pièce de tête (106) pour transmettre des signaux de stimulation et de la puissance par l'intermédiaire de la bobine émettrice-réceptrice externe (130) à la bobine émettrice-réceptrice d'implant (132), et le composant externe (106) étant connecté à un processeur sonore (104) configuré pour générer des signaux de stimulation.
